Europäisches Patentamt

European Patent Office

Office européen des brevets

Publication number: **0 275 705**
**A2**

# EUROPEAN PATENT APPLICATION

(21) Application number: 87311402.9

(22) Date of filing: 23.12.87

(51) Int. Cl.4 **C12P 21/00** , **C07K 15/00** ,
**G01N 33/574** , **G01N 33/577** ,
**C12N 5/00**

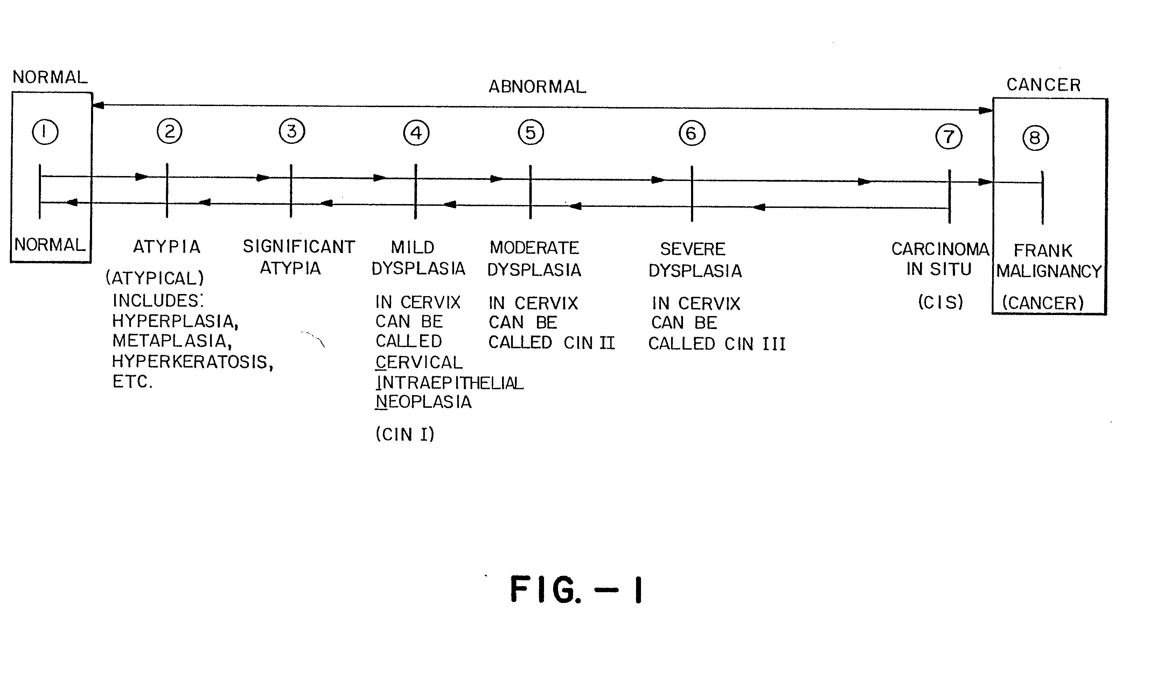

A request for addition of a sheet with drawing (Fig.1) has been filed pursuant to Rule 88 EPC.

(30) Priority: 23.12.86 US 946237

(43) Date of publication of application:
27.07.88 Bulletin 88/30

(84) Designated Contracting States:
AT BE CH DE ES FR GB GR IT LI LU NL SE

(71) Applicant: InTek Diagnostics
1450 Rollins Road
Burlingame California 94010(US)

(72) Inventor: Liu, Y. S. Victor
490 Mill Stream Drive
San Leandro California 94578(US)
Inventor: Yonkovich, Shirlee J.
165 East O'Keefe
Menlo Park California 94025(US)
Inventor: White, Carmen F.
11 Mirabel Avenue
San Francisco California 94110(US)
Inventor: Gottfried, Toby D.
10 Rustic Way
Ordina California 94563(US)
Inventor: Ranken, Raymond R.
1051 Beach Park Boulevard, No. 302
Foster City California 94404(US)

(74) Representative: Bizley, Richard Edward et al
BOULT, WADE & TENNANT 27 Furnival Street
London EC4A 1PQ(GB)

(54) **Monoclonal antibodies specific for human basal cells, SCC and precancerous cells.**

(57) Monoclonal antibodies specific for an antigen site associated with (a) the intra-cellular portion of human basal cells and human malignant squamous carcinoma cells, and (b) morphologically normal, non-basal squamous epithelial cells which are pre-cancerous. The method of producing the antibodies. Also, such antibodies in a diagnostic test kit.

# MONOCLONAL ANTIBODIES SPECIFIC FOR HUMAN BASAL CELLS, SCC, AND PRE-CANCEROUS CELLS

In 1975, Köhler and Milstein developed a lymphocyte fusion method for the production of homogeneous and highly specific monoclonal antibody in unlimited quantities (G. Köhler and C. Milstein, Nature 265, 495, 1975). In general, monoclonal antibodies are produced by fusing mouse myeloma tumor cells to spleen cells derived from a mouse that has previously been immunized with a foreign substance (antigen). The derived hybrid cells, called hybridomas, carry the desired parental traits, i.e., vigorous growth in tissue culture derived from the myeloma tumor cell and antibody production from the splenic B cell. The exquisite specificity, unlimited supply of homogenous reagent and its advantage of being able to use impure and unknown antigens to produce pure antibody reagents have revolutionized the cancer research field by providing an unprecedented tool for cancer diagnosis and therapy.

A significant amount of work has been done to adapt hybridoma technology to the cancer area. The present invention focuses on monoclonal antibodies capable of binding to a tumor of the human squamous epithelial cell systems, i.e., squamous cell carcinoma (SCC).

The transformation of normal cells to tumor cells is called neoplastic development. This process can be divided into at least three phases -- initiation, promotion and progression. Initiation involves the conversion of normal cells to latent tumor cells. Promotion is the stimulation of latent tumor cells to form tumors (neoplastic evolution).

Figure 1 illustrates a summary scheme of malignant transformation of normal squamous epithelial cells through a variety of abnormal conditions, as indicated by (2) to (6). Each stage exhibits greater deviation from the normal, eventually resulting in frank malignancy. Transformation can be evolved by jumping out of sequence of events.

The morphologically distinct lesion that arises during the course of carcinogenesis are usually referred to as hyperplasia, dysplasia, metaplasia and hyperkeratosis, etc. and are generally believed to represent precursor lesions for neoplasms. The cell populations present in these lesions may display a number of morphologic and functional abnormalities. After the evolution of a neoplasm, progression results in the acquisition of qualitatively new properties by the neoplastic cells, endowing them with greater abnormalities. The difference between benign and malignant neoplasms is that the latter is capable of invasion and metastasis. Benign neoplasms generally exhibit a small tendency to progress to malignancy. This suggest that the two courses of development may be separate and signal a potential distinction between pre-cancerous and pseudo-cancerous stages.

Pathologists have given descriptive terms and evaluated dysplastic lesions by both light and electron microscopy. Using this subjective criteria, they have identified mild, moderate and severe dysplasia, carcinoma in situ and invading frank malignancy. These terms are universally accepted in gynecological pathology. Similar pathologies are found in the lung, esophagus, oral mucosa, breast, endometrial, ovary and prostrate, yet there are no universally accepted descriptions of these lesions. However, the descriptions cannot differentiate between lesions which will progress to cancer and those that will remain benign. To date, pathologists have no method of accurately identifying these pre-cancerous cells. The development of an antibody reagent capable of identifying morphologically normal or morphologically atypical cells that will progress to malignancy would revolutionize cancer diagnosis and therapy, providing screening procedures for early detection. Conventional medical procedures could then be used with a significantly higher rate of success.

Several monoclonal antibodies have been reported in various publications to be able to bind to human squamous cell carcinoma; such antibodies have, in general, only been tested with a relatively small number of clinical specimens.

The following Table 1 summarizes certain information regarding such monoclonal antibodies. Table 1 includes: (a) publications regarding the antibody; (b) antibody class or subclass; (c) immunization protocol for the creation of the hybridoma; (d) screening protocol for the selection of useful hybridomas; (e) performance of the antibody; and (f) comments re utility of the antibody.

Table 1

A. Name of Monoclonal Antibody: VM-2

Publication:
V.B. Morhenn, et al., J. Clin. Invest. 76, 1978, 1985

Class of Subclass of Antibody:
IgG1

Immunization Protocol:
Human skin cells from psoriatic plaques. (Note: immunogen is not derived from cancer.)

Screening Protocol:

Primary screening used immunofluorescence on normal skin and psoriasis.

Performance of Antibody:

1) In human normal squamous epithelial tissues, binds to the basal cells and basement membrane (kidney glomeruli) and suprabasal cells.

2) High sensitivity toward squamous cell carcinoma (97%).

3) Does not bind to cytokeratin.

4) Does not have information on non-squamous cancer cell line such as HTB113. Does not bind to lymphoma cell lines.

5) In cervical dysplasias, stained 19 out of 21 (90.5%) of moderate dysplasia, 12 out of 12 (100%) of severe dysplasia.

6) No information on retrospective, sequential specimens.

Comments re Utility:

Cannot distinguish between benign and pre cancerous squamous epithelial cell transformation.

B. Name of Monoclonal Antibodies: AE-1, AE-2 and AE-3

Publications:

T. T. Sun, et al., J.Invest. Dermatol. 81, 1095, 1983; and J. Reibel, et al., Acta. Path. Microbiol. Immunol. Scand. Sect. A, 93, 323, 1985.

Class or Subclass of Antibody:
IgG1

Immunization Protocol:
Immunized with sodium dodecyl sulfate (SDS) denatured keratin isolated from human callous.

Screening Protocol:
Primary screening used ELISA on keratins from callous.

Performance of Antibodies:

1) In human normal squamous epithelial tissues, antibody AE-1 only binds to the cells in basal layer, antibody AE-2 binds only to the cells in suprabasal layers but not cells in the basal layer, antibody AE-3 binds to the whole thickness (all layers, including basal cell layer) of the epithelia.

2) In normal esophageal squamous epithelial tissue, antibody AE-1 binds to the entire thickness of the epithelia.

3) In breast infiltrat ing ductal adenocarcinoma, antibody AE-1 binds to the tumor cells.

4) Antibody AE-1 stains all squamous cell carcinoma in oral cavity. Staining pattern is not homogeneous but highly variable and randomly distributed. Antibody AE-2 staining for SCC is also random, totally negative in two SCC's, in other SCC's only few discrete cells were positive. No information on antibody AE-3 alone on SCC's.

5) All three antibodies bind to different classes of cytokeratins.

6) No information on these three antibodies on cervical atypical or dysplastic tissues or cells. Variable and random staining patters were seen by antibodies AE-1 and AE-2 on oral atypical and dysplastic tissues. No retrospective, sequential specimen information.

Comments re Utility:

1) Positive identification of normal and abnormal epithelial cells.

2) Distinguish tumor of epithelial versus non-epithelial origin (i.e., carcinoma versus non-carcinoma).

C. Name of Monoclonal Antibodies: PKK1 and PKK2

Publications:

Holfhofer, et al., Lab. Invest. 49, 317, 1983 and product inserts of antibodies PKK1 and PKK2 from Labsystems, Inc. Chicago, IL.

Class or Subclass of Antibodies:
IgG

Immunization Protocol:
Immunized with cytoskeletal proteins isolated from pig kidney epithelial cell line.

Screening Protocol:
Primary screening used ELISA on proteins and cell lines.

Performance of Antibodies:

1) In human normal squamous epithelial tissues, antibody PKK1 binds to cells in all layers of the stratified system, while antibody PKK2 binds to the cells in basal layer and para-basal layers in normal stratified squamous epithelial tissues.

2) Antibody PKK2 binds to columnar epithelial tissue in normal lung.

3) Antibody PKK2 binds to SCC.

4) Antibody PKK2 binds to bronchial alveolar carcinoma.

5) No information on atypical, abnormal squamous epithelial tissue information.

6) PKK1 and PKK2 binds to cytokeratins.

Comments re Utility:

Distinguished tumor of epithelial versus non-epithelial origin.

D. Name of Monoclonal Antibody: MAK-6

Publication:

Production data of Monoclonal Cocktail MAK-6™, from Triton Bioscience, Inc., Alameda, CA.

Class or Subclass of Antibody:

IgG

Immunization Protocol:

Component antibody KA-4 is immunized with human sole epidermis callous. Component antibody UCD·PR10.11 is immunized with shed extra-cellular antigen from human breast cancer cell line MCF-7.

Screening Protocol:

Component antibody KA-4 is screened on normal and tumor breast tissue. Component antibody UCD·PR10.11 is screened on tissue culture supernate of breast cancer cell line MCF-7.

Performance of Antibody:

1) Stains tissues contain cytokeratins and other filament types: vimentin (mesenchymally derived), desmin (muscle, hyogenic cells), glial fibullary acidic protein (astroglia and other glial filaments) and neurofilament proteins (neuronal cells).

2) There is no intensive information on MAK-6 monoclonal cocktail's staining on SCC, atypical and dysplastic tissues.

MAK-6 cocktails binds to cytokeratins.

Comments re Utility:

Distinguish tumor of epithelial origin (i.e., carcinoma versus non-carcinoma).

E. Names of Monoclonal Antibody: A-9

Publication:

K. A. Kimmel and T. F. Carey, Cancer Res., 46, 3614, 1986.

Class or Subclass of Antibody:

IgG2a

Immunization Protocol:

Immunized with cancer cell line UM-SCC1, a cell line derived from tumor of SCC of floor of mouth.

Screening Protocol:

Primary screening used headmadsorption assay on squamous cell carcinoma cancer cell lines.

Performance of Antibody:

1) In normal human squamous epithelial tissue, binds to basement membrane and cells in the basal layer.

2) Stains only the proliferating and invading cells of SCC.

3) Does stain some non-SCC epithelial cancer cell lines.

4) Does not stain all SCC cancer lines.

5) No information on atypical, dysplastic abnormal tissues or cells.

Comments re Utility:

May be a tumor marker for metastasis SCC.

F. Name of Monoclonal Antibody: SQM1

Publication:

K. Boeheim, Int. J. Cancer, 36. 137, 1985.

Class or Subclass of Antibody:

IgM

Immunization Protocol:

Used cancer cell line SCC15 derived from human SCC of the tongue as immunogen.

Screening Protocol:

Primary screening used SCC cancer cell lines.

Antibody Performance:

1) Antibody does not bind to basement membrane and does not bind to cells in the basal layer in normal human squamous epithelial tissue.

2) Antibody stains SCC of head and neck (17 18 positive, ≥94%).

3) Antibody stains breast cancer cell line MCF-7.

4) There is no information on antibody reactivity toward atypical or dysplastic abnormal tissues or cells.

Comments re Utility:
May be useful for head and neck SCC diagnosis.

G. Name of Monoclonal Antibody: 99-57

Publication:
I. Koprowska, et al., Cancer Res. 45, 5964, 1985.

Class or Subclass of Antibody:
IgG1

Immunization Protocol:
Immunized with a bladder carcinoma cell line, T-24.

Screening Protocol:

1) Primary screening used immunoperoxidase on cervical cancer cell line, SW576.
2) Also screened on normal cervical smears.
Performance of Antibody:

1) Stains only less than 37% of cervical SCC.
2) Stains 0/2 cervical CIN I, 0/1 cervical CIN II, 4/13 cervical CIN III.
3) No information on SCC other than cervical area.
Comments re Utility:
Sensitivity toward SCC is too low to have clinical value.

H. Name of Monoclonal Antibody: PF PF1 A,B,C,D,E
Publication:
P.P. Fernsten, Cancer Res. 46, 2970, 1986

Class of Subclass of Antibody:
IgG3

Immunization Protocol:
Squamous cell carcinoma cell line derived from lung, USCLS-1.

Screening Protocol:
Primary screening used ELISA on cancer cell lines and frozen sections of tumor and normal tissues.

Performance of Antibody:

1) Antibodies do not stain basement membrane and cells in the basal layer in human normal squamous epithelial tissue.
2) Antibodies stains SCC and adenocarcinomas.
3) No information on atypical and dysplastic abnormal tissues or cells.
Comments re Utility:
Antibodies have been proposed for therapeutic use.

I. Name of Monoclonal Antibody: Ca1

Publications:
F. Ashall, et al., Lancet ii, 1, 1982; A.H.M. Shabana, et al., Br. J. Cancer, 48, 527, 1983; and B. Czerniak, et al., Cancer, 55, 2783, 1985.

Class or Subclass of Antibody:
IgM

Immunization Protocol:
Immunized with glycoproteins from laryngeal cancer cell line, H. Ep.2.

Screening Protocol:
Primary screening used laryngeal cancer cell line, H. Ep.2.

Performance of Antibody:

1) Randomly stains SCC, adenocarcinoma and mesothelioma, normal tissue, head and deck dysplasia.
2) Antibody CA1 is not specific enough to be of value in the diagnosis of cancerous and precancerous lesions in oral cavity.
Comments re Utility:
May be useful for the detection of mesothelioma.

J. Name of Monoclonal Antibodies: CK1-CK-4

Publication:
E. Debus, et al., The EMBO Journal 1, 1641, 1982.

Class or Subclass of Antibodies:
IgG1

Immunization Protocol:
Immunized with cytoskeletal preparation from human cervical cancer cell line, HeLa.

Screening Protocol:
Primary screening used immunofluorescence on HeLa cells.

Performance of Antibodies:

1) Antibodies stain all squamous cell carcinoma.

2) Antibodies stain cytokeratin.

3) Antibodies also stain adenocarcinoma.

4) Antibodies stain almost all epithelia from the body.

5) No information on atypical or dysplastic abnormal tissues or cells.

Comments re Utility:
Distinguish tumor of epithelial versus non-epithelial origin (i.e., carcinoma versus non-carcinoma).

K. Name of Monoclonal Antibody: CAR-3

Publication:
M. Prat, Cancer Res., 45, 5799, 1985.

Class or Subclass of Antibody:
No information

Immunization Protocol:
Immunized with paraformaldehyde fixed human epidermoid lung carcinoma cell line, A 431.

Screening Protocol:
Primary screening used ELISA on cancer cell lines.

Performance of Antibody:

1) Antibody binds to SCC and adenocarcinoma.

2) Antibody also binds to normal epithelium.

3) No information on atypical or dysplastic abnormal tissues or cells.

Comments re Utility:
Distinguish tumor of epithelial versus non-epithelial origin (i.e., carcinoma versus non-carcinoma).

Although the effective dates of some of the above references of Table 1 are not early enough to constitute prior art against the claims of this application, they are included for general background purposes.

The antibodies of the present invention are focused to identify and diagnose squamous cell carcinoma and its pre-cancerous precursors in the head and neck region, oral mucosa, gynecological region, lung and SCC involved lymph nodes.

The head and neck region is particularly demanding in diagnosis because it requires a three dimensional interpretation. In most instances, the surgeon removes tissues or organs with well defined boundaries, for example, the uterus or a lobe of the lung. The interpretation of histological data generated from tissues submitted from the oral cavity or pharynx may at times be very difficult. The topography of the cylindrical pharynx as it emerges from the oral cavity where it splits to form the esophagus and trachea has confusing elements to the interpretation margin. Specimens shrink during fixation and processing providing some elements of distortion to the pathologist. Contraction of normal tissue overlying the tumor may make margins positive because of varying degrees of tissue shrinkage.

The most common cancer of the head and neck is SCC. Four principal types of errors are possible in frozen section diagnosis; sampling, interpretation, communication, and technical. Interpretation errors are those made when the pathologist fails to recognize a lesion that is actually present in the frozen section. In tissues of the head and neck, this error is not uncommon in the interpretation of cites of the previous laryngeal biopsy, in irradiated tissues and in unsuspected unusual lesions. Lack of identification of the exact source of the tissue specimen submitted for frozen section diagnosis is a common error of communication. Technical factors, e.g. slides of poor quality, lead to errors in interpretation by the pathologist.

The absence of positive margins in the head and neck region does not guarantee local control of the disease, nor is it a reliable guide to the biological behavior of the tumor. However, the correlation between the positive frozen section margins in patient survival is very significant, since, typically, the patients succumb to their disease within a relatively short time.

The most common type of oral cancer is SCC which accounts for about 90% of all oral malignancy. (Oral Cancer 1985 Ed. S. Silverman, American Cancer Society, New York) Less than half of the patients with oral cancer are cured. (W.H. Binnie and K.V. Ranken, J. Oral Pathol. 13, 333, 1984) Even among cured patients, there may be severe degrees of dysfunction and disfigurement. Currently there is difficulty in the diagnosis of oral lesions because of the oral appearance of the oral cavity of ill-defined, variable, controversial and poorly understood lesions. (J.J. Pindborg, Surgical Pathology of the Head and Neck, Vol. I, Ed. L. Barnes, Ch. 7, 279, Marcel Dekker, Inc., New York; Oral Cancer, 1985 Ed. S. Silverman, American Cancer Society, New York). Most of these are benign but may present changes that are easily interpreted as malignancy. (J.C. Adrian, Oral Surg., 57, 625, 1984) There are other lesions which are in the early stages of malignancy and are mistaken for a benign mucosal alteration. (M.N. Chau and B.G. Radden, J. Oral Pathol. 13, 546 1984). The management of oral lesions that may not remain benign is a significant problem because diagnostic procedures which require follow-up biopsies of the tongue or larynx, for example, can be costly, disfiguring and lead to dysfunction.

Leukoplakia, erythroleukoplakia or any suspect lesion is routinely biopsied and evaluated for the presence of atypia, dysplasia and malignancy. This evaluation is now carried out by the pathologist in the subjective microscopic analysis of tissue pattern and cell morphology on hemotoxlin and eosin stained tissue sections. Epithelial changes resulting in patterns which are not normal but clearly not malignant are termed either atypia or dysplasia. Leukoplakia and erythroleukoplakia are categorized as "premalignant lesions" with some types of evaluated as dysplastic. Frank malignancies are readily diagnosed but premalignant or pre-cancerous lesions are diagnosed with difficulty and inconsistencies. The interpretation and grading of dysplastic epithelium in pre-cancerous lesions is subjective and therefore, variable. Since some dysplasia will progress to invasive carcinoma, it is important that this potentially hazardous lesion be accurately diagnosed and treatment initiated immediately.

A traditional histological method of grading dysplastic lesions does not provide a prognosis for the transformation to invasive squamous cell carcinoma. Although morphologically dysplastic leukoplakia is generally thought to be the beginning stage in the spectrum of developing invasive squamous cell carcinoma, malignancies can arise from lesions such as benign hyperkeratosis, Lichen Planus and chronic candidiases. There is no reported way to determine and predict which lesions will progress to invasive squamous cell carcinoma. There are no reliable histochemical or biochemical methods for testing the malignant potential of human musocal lesions.

Leukoplakia is a term used to designate a clinical white patch or plaque on the oral mucous membrane that cannot be removed by scraping and cannot be classified clinically or microscopically as another disease entity. Most of these lesions are a reflection of benign hyperkeratosis. Silverman and co-workers studied and followed patients who presented with leukoplakia for a mean of 7.2 years. The primary biopsy of 257 lesions revealed 235 patients with benign hyperkeratosis and 22 primary biopsies showed some degree of epithelial dysplasia. Forty-five patients (17%) from among the total number of patients subjected to biopsy subsequently developed SCC. Eight of these occurrences were from dysplastic lesions. This study confirms that leukoplakia may present a pre-cancerous lesion. However, clinical or microscopic characteristics seldom appear that allow the clinician to identify reliably which hyperkeratoic lesion will transform into carcinoma.

Leukoplakia that clinically has an erythematous component (white plaques or nodules speckled on a reddened mucosa) is associated frequently with the microscopic changes of carcinoma, carcinoma in situ, or epithelial atypia. The red (erythroplakia) or red and white (erythroleukoplakia) oral changes are far more likely to manifest a dysplastic or malignant change or to transform into these pathologic states than solely white (leukoplakia) lesions.

Exfoliative cytology, the examining of isolated cells or clumps of cells obtained from scraping or washing of mucous membranes or tissues from various body sites, such as cervix, vagina, endometrium, oral cavity, lung and skin, etc., has long been a useful procedure in pathological examination. With the advent of the Papanicolaous examination of cervical cytological smears, (G.N. Papanicolaou, Science, 95, 438, 1942), the incidence and mortality rate of invasive carcinoma of the cervix have reduced dramatically. (W.B. Jones and P.E. Saigo, CA-A Cancer J. Clinicians 36, 237, 1986). Along with declining incidence rates for invasive cervical cancer, there has been a marked increase in pre-invasive disease. In the U.S., the number of women who have an early cervical cancer precursor is estimated to be more than four times the number of those with in situ carcinoma (W.T. Creasman, Comtemp. Obstet. Gynecol. 21, 53, 1983) i.e., more than 180,000 cases of atypical or dysplastic lesions. When a satisfactory Papanicolaou smear is reported to contain only normal cells in a patient with healthy cervix, the smear is usually repeated at recommended intervals. When the smear contains cells described as suspicious for malignancy, colposcopic examination and biopsy are required. The dilemma arises when laboratories utilizing the Papanicolaou classification group together within the same class a wide rate of cytologic abnormalities that may signify inflammatory atypia, neoplasia, or, in some cases, diagnostic uncertainty. Although dysplasia is generally thought to be the beginning stage in the spectrum of developing squamous cell carcinoma, it is still impossible to determine and predict which lesions will become invasive (pre-cancerous) and which will not (benign). Because such findings are often all reported as a class II (atypical) smear, some patients with pre-invasive disease, or even invasive cancer of the cervix, may go unrecognized. This can occur in the patient with a histologi- cally early lesion where the associated cytologic abnormality may be minimal and difficult to interpret, as well as in the patient with a more advanced lesion, where the sample contains only a small number of atypical cells not representative of the significant underlying abnormality. Because the Papanicolaou classification fails to convey the level of significance of atypical cells, it is viewed as being inadequate for reporting such findings.

The present invention is concerned with certain diagnostic methods using novel monoclonal antibodies specific for an antigenic site on human malignant squamous cells and their pre-cancerous precursor cells. The method of the invention finds utility in the detection of squamous cell malignancy in the cervix, vagina, uterus, bronchus, esophagus, oral cavity, head and neck and skin, etc., and exfoliative cells aspirated from tumors, tissues, biopsied clinical specimens including lymph nodes for an antigen antibody immunologically specific, non-subjective evaluation.

The present invention relates to monoclonal antibodies and fragments specifically reactive with an antigenic site associated with, and preferably found in, normal human basal cells, human pre-cancerous squamous epithelial cells and frank malignant squamous carcinoma cells, but not with benign squamous epithelial cells. The antigenic sites are preferably found in such cells.

As used herein, the term "pre-cancerous" refers to squamous cells which are committed to go to invasive SCC in any stage of transformation ("pre-SCC stage) which is prior to cells in SCC frank malignancy (cancerous cells). The pre-SCC stage is a stage which includes squamous cells which are morphologically normal, atypia (hyperplasia, metaplasia, hyperkeratosis), significant atypia, dysplasia (mild dysplasia - CIN I, moderate dysplasia - CIN II, and severe dysplasia - CIN III) or carcinoma in situ (CIS). The term "pre-cancerous" is to be distinguished from "benign", defined as a type of squamous cells in the pre-SCC stage not committed to go to invasive SCC.

Preferably the antibody of the present invention stains with a high degree of sensitivity, e.g. over 90% SCC of the lung. On the other hand, it does not strain adenocarcinoma or small cell carcinoma of the lung. Similarly, it stains SCC of the cervix but not adenocarcinoma of the endocervix, endometrial or ovarian origin. As confirmation that the antibody stains only those pre-SCC squamous cells which are pre-cancerous, the antibody only stains on the order of 40% or less of dysplastic cells. The antibody further stains essentially all of the SCC in the head and neck region. In contrast, the antibody does not stain benign cells in the following areas; adrenal, aorta, brain, breast, bladder, colon, heart, kidney, liver, lymph node, ovary, pancreas, prostate, spleen, stomach, thyroid, and only a trace in muscle tissue.

With respect to normal lung tissue, it stains only the normal basal cells but not normal columnar alveolar cells, macrophages or the like. With respect to gynecological and head and neck cells, it also stains only the basal cells of the epithelium but not the stratum cornium, superficial, intermediate, parabasal, or other cells of the epithelium.

The antibody preferably is produced by a hybridoma formed by fusing with other cells, antibody-producing cells derived from an animal, specifically mouse, immunized with tissue or tissue preparations, rather than in vitro culture derived cancer cell lines from specific sources. The source should include human cancer squamous cell tissue, and/or tissue containing normal basal cells. It may include, in addition morphologically normal, atypical, or dysplastic, non-basal human epithelial cells. The spleen cells from the immunized animal are fused with non-immunoglobulin secreting myeloma cells and a hybridoma is selected which secrets a hybridoma antibody with the indicated specificity. The aforementioned technique of Köhler and Milstein may be used.

The invention also relates to a method of using such antibody for determining the presence of a pre-cancerous condition in normal, abnormal or dysplastic, non-basal human squamous epithelial cell specimens. Such specimens may originally include exfoliated cells from sources such as vaginal, cervical, an endometrio-uterine smears, bronchial secretions such as sputum and bronchial washes, biopsy specimens, surgically removed tissues, cytological aspirates and the like.

In a preferred embodiment, the monoclonal antibody of the present invention is capable of detecting a site on an antigen associated with (a) normal human basal squamous epithelial cells, (b) human SCC, and/or with (c) pre-SCC stage (morphologically normal, atypical or dysplastic) human non-basal, squamous epithelial cells which are pre-cancerous, but not found in those cells which are benign.

More specifically, the present invention relates to such monoclonal antibodies of the IgG or IgM type which do not recognize at least 98% (substantially all) of specimens comprising morphologically normal, non-basal squamous epithelial cells. On the other hand, the antibody is specific for a site on an antigen with at least some (e.g. 1% or less) of morphologically normal, non-tumor, non-basal, human squamous epithelial cell specimens. The percentage of non-basal cells that the antibody recognizes increases from a relatively low percent at the normal stage, e.g. less than 2% through the atypia and into the dysplasia stage into the carcinoma in situ (CIS) stage until frank malignancy recognizes well over 90% of the specimens. For example, in the atypia stage it may recognize on the order of 1% and in the dysplasia stage it may recognize on the order of 3% to 40% in the cervix and head and neck region.

The above combination of properties of the monoclonal antibody of the present invention is indicative that the antibody is specific for a site on an antigen in pre-cancerous cells at the pre-SCC stage, or for cancerous cells, and for basal cells but not for benign cells.

Another characteristic of the antibody is that at least some, if not all, the antigen which it recognizes is found in the intra-cellular portion of the cells. That is, at least a portion of the antigen is in the cells. More typically, the antigen is found in the cell cytoplasm. Thus, whether it recognizes the normal, abnormal or cancerous cells, some of the antigen is in the intra-cellular portion, and not on the cell surface also. However, in some cells, the antigen may be found in both the cytoplasm and projecting through the cell membrane.

In a preferred embodiment, the antibody has been found to recognize an antigen which is a component of a macromolecule which contains protein. Furthermore, it has been found to be stable in the presence of non-ionic detergent Triton X-100, or in periodic acid or neuraminidase treatment, and appears bright diffuse and bright fibrillar homogeneous by immunofluorescence or immunoperoxidase staining. In addition, the antigen which it recognizes resides in the cytoskeletal elements of intermediate filaments by electron microscopic indirect colloidal gold particles (10 nm) staining. The antibody is of either the IgG or IgM class.

The invention also relates to various known general techniques for diagnosis using antibodies, specifically monoclonal antibodies, and adapting them to the unique properties of the antibodies of the present invention. In one method, cells in a specimen suspected of being cancerous or precancerous are contacted with the monoclonal antibody which is capable of distinguishing benign, non-basal, squamous epithelial cells from pre-cancerous, pre-SCC stage cells and from cancerous cells. Contact is performed under well known conditions for binding of the antibody to the antigen in the specimen to form immune complexes. The presence or absence of binding is determined by conventional techniques using a "detection system". In one embodiment, the antibody is conjugated with a label of a conventional type such as radioisotope, enzyme, fluorochromes or a metal complex label to directly stain the specimen. Conjugates of antibodies with such conventional labels are well known.

Method of binding labels to antibodies and antibody fragments are well-known. See, e.g. U.S. patent nos. 4,220,450, 4,235,869, 3,935,074, 3,996,345 and 3,817,837.

The detection system may also include other immunological components, specifically another antigen or antibody, reactive with the monoclonal antibody or antibody fragment, and conjugated with a detectable label. Then, the label is observed and detected.

In some detection systems, such as the enzyme system, additional components are used, such as substrates, or chromogens, to render the label detectable. Furthermore, these labels may be visible to the naked eye or require external excitation and reading such as fluorescence or radioactive labels. In other detection systems, the first component is an immunological substance such as another antibody reactive with the monoclonal antibody or antibody fragment which is reactive with the monoclonal antibody and conjugated to a reactive substance such as avidin and biotin. Here, a second component may be used which is a conjugate of (a) a substance reactive with the first component, (e.g. biotin which is reactive with the avidin) or (b) a detectable label such as a fluorescent label. This is an indirect labeling technique.

The specimen containing the antigen to be detected may be from any of a variety of locations on or within the body. The method is useful for detecting a pre-cancerous or cancerous condition in human squamous epithelial cells or tissues in uterine, cervix, vagina, oral mucosa, head and neck, skin, lung, esophagus and bladder in the surface of biopsy specimens, especially lymph nodes, and from body fluids, such as sputum, bronchial washes, urine, semen, blood and ascites.

A monoclonal antibody particularly useful in the method of the present invention is a novel antibody designated MAb 17.13.Cl.10 (herein the 17.13 antibody). It was derived from the fusion of splenocytes from female BALB/c mice immunized with a poorly differentiated human larynx squamous cell carcinoma tissue (containing normal and abnormal transformation tissues) with non-secreting mouse myeloma SP2/0. It is an IgM(kappa A) immunoglobulin.

A permanent deposit of a viable cell line producing the 17.13 antibody is maintained at the ATCC under number HB9294.

The screening protocol was as follows. Primary screening used immunofluorescence or immunoperoxidase testing on human frozen tissues, not in vitro derived cancer cell lines. Tissues screened consist of tumor, normal and abnormal specimens. Screening also composed of exfoliated human cells from cervix, lung, oral cavity and esophagus.

The 17.13 antibody has demonstrated extremely high tumor association with squamous cell carcinoma. To date, it is 99% sensitive for SCC in the head and neck (91.92 positive); 100% sensitive for SCC in the cervix (20.20 positive) and 96% sensitive for SCC in the lung (25.26 positive).

The 17.13 antibody does not stain normal non-basal, squamous epithelial cells and tissues. In extensive screening of normal cervical cell smears and human autopsy panels, it did not demonstrate staining except for normal epathelial basal cells. Furthermore, it has not demonstrated staining for red and white blood cells and lymph nodes hyperplasia. On flow cytometry screening, the 17.13 is negative on T cell lymphoma and Burkitt's lymphoma cell lines.

In general, the above testing was performed using the 17.13 antibody in a sensitive indirect immunoperoxidase technique. Specifically, after reaction with the specimen, the 17.13 bound antibody was reacted with a secondary biotin-conjugated goat anti-mouse IgM antibody. This, in turn, is followed by an avidin-biotin horseradish peroxidase complex that binds specifically to the avidin conjugated secondary antibody. Visualization of the total complex is achieved when diaminobenzadine (DAB) and hydrogen peroxide are added. An insoluble brown precipitate is deposited at the site of 17.13 antibody binding.

In summary, the 17.13 antibody performs as follows. In human normal squamous epithelial tissue, it only binds to the cells in basal layer. In human normal squamous epithelial tissues, it does not bind to basement membrane, or cells in intermediate or suprabasal layers. It has high sensitivity toward squamous cell carcinoma ( 97%), in SCC, binds to the whole thickness of the squamous epithelial sheath, stains tumor cells in homogenous bright pattern. It does not bind to cytokeratins or non-squamous cancer cell lines such as HTB113 (a human endometrial adenocarcinoma), T cell lymphoma or Burkitt's lymphoma. In atypical and dysplastic abnormal tissues or cells, it only stains 40% or less of the specimens tested. In retrospective, sequential specimens, it stained the abnormal specimens which eventually have transformed to cancer. In competitive studies, the staining intensity and pattern do not reduce or alter by other antibodies tested.

The invention is also applicable to binding fragments of the monoclonal antibody such as Fab, F-(ab)₂, Fv, and the like. The techniques of forming the antibody fragments are well known. For example, binding fragments may be prepared by peptide digestion of the antibody using proteolytic enzyme such as papain or pepsin.

While the antibody is of the IgM class from a murine source, it is also applicable to other antibodies which are "functionally equivalent" with the above antibody or from other sources than a murine source such as human, rabbit, goat and guinea pig as well as another classification such as IgG and IgA and the like. A "functional equivalent" is an antibody capable of binding to the above specified antigenic site and capable of competing with the 17.13 antibody for such site. Such antibody will bind to such antigenic site and block the 17.13 antibody from binding to such site.

In another aspect of the invention, the present antibody is combined into a cocktail with other antibodies which may recognize sites of other antigens which can also be associated with pre-cancerous and cancerous cells but not with benign, non-basal, squamous epithelial cells. In this manner, if the antigen for which the present antibody is specific is not detected, the other antibodies may detect the antigen as an indication of a cancerous or pre-cancerous condition.

The invention is also applicable to therapeutic and in vivo imaging uses of the monoclonal antibodies of the present invention. In therapeutic use the antibody could be bound conjugated to a substance of high toxicity for SCC, such as radionuclides or toxins to preferentially seek out the cancerous and pre-cancerous cells. For in vivo imaging, it can be bound to labels or markers such as the foregoing ones used for diagnostics for preferential imaging of the same types of cells.

Specific examples of the practice of the present invention are set forth in the following examples by way of illustration.

Example 1

The formation of the 17.13 antibody is as follows:

A. IMMUNIZATION PROCEDURE

A female BALB/c mouse was immunized exclusively with cells obtained from the tissue digestion of a squamous cell carcinoma supraglottic larynx tumor. The mouse was bled prior to immunization to acquire a control serum sample.

The primary immunization was a 10⁶ cell/mouse I.P. injection of fresh tumor digest suspended in P.B.S. Twenty-four days later the mouse was challenged with a subcutaneous injection of 10⁶ cells. Eight days following the challenge boost a serum sample was collected to evaluate the immune response. Prior to fusion a consecutive three day challenge was initiated. The first immunization of this challenge was $10^6$ cells in P.B.S. administered subcutaneously. The final two challenges were given I.P. and consisted of $5 \times 10^5$ cells/injection.

## B. FUSION PROTOCOL

A highly squamous cell carcinoma cell associated antibody (MAb 17.13.C1.10) was prepared from fused murine spleen cells and SP2/0 cells in accordance with the procedure of Kohler and Milstein.

The mouse was bled by incision of the brachial artery and immersed in 70% ethanol, placed on a clean surface, and the spleen was removed aseptically. The spleen was rinsed in a petri dish with about 10 ml of PBS/G and 20 ml of an antibiotic and antimycotic solution. The spleen was placed in a second PBS solution containing antibiotic and antimycotic, and the spleen cells were removed, centrifuged, resuspended in PBS/C and recentrifuged.

The separated spleen cells resuspended in PBS/G were combined with SP2/0 cells in a 1:1 ratio in a 50 ml tube, centrifuged, and 1-2 ml of PEG (mw 1300-1600) at 37°C were added over 1.5 minutes to the pellet with stirring. The tube was placed in a 37°C waterbath for 90 seconds, swirling the cells. The PEG was slowly diluted by adding PBS/G dropwise over 1 minute, adding 5 ml of PBS/G slowly over 2 minutes, adding 30 ml of PBS/G over 5 minutes, and then completely filling the tube with PBS/G, letting the final mixture stand for 5 minutes.

The suspension was centrifuged at 200 g for 10 minutes, discarding the supernatant. The pellet cells were blasted with 50 ml of RPMI medium containing 10% fetal calf serum with an autopipettor, and the cell suspension was centrifuged at 200 g for 10 minutes. The cells were then gently resuspended in HAT media by blasting the pellet with media from an autopipettor. Sufficient HAT media was added to dilute the pellet to a concentration of $10^6$ spleen cells per ml. The cells were plated at 100 microliters per well into 96 well microtiter plates.

In this manner, a spleen having approximately $2 \times 10^8$ cells was resuspended after fusion in 200 ml HAT medium and distributed over approximately 2000 wells (10 microtiter plates). The plates were incubated at 37°C in a humidified incubator containing 5% $CO_2$ and 95% air.

## C. PURIFICATION

Hybridomas were grown as ascites tumors in pristane-primed BALB/c mice. Pristane (tetramethylpentadecane) was injected in volumes of 0.5 ml i/p., one week prior to i.p. injection of $10^7$ live hybridoma cells per animal. Ascites fluid was drained 1 to 2 weeks later by abdominal puncture and was used as a source for antibody purification. The IgM fraction of ascites fluids was purified by gel filtration chromotography (LKB Ultrogel AcA-22). The IgM fraction was eluted with 0.1M TRIS-HCl ph 8.0, 0.15M NaCl containing 0.001% merthiolate (TRIS-SAL Buffer). The purity was determined by SDS gel electrophoresis and staining with Coomassie blue using densitometer analysis. Characterization of MAB 17.13.C1.10 specificity was by immunohistology and immunocytology methods using indirect immunofluorescence and immunoperoxidase staining procedures. Antibody typing for mouse isotype and light chain specificity was by Ouchterlony immunodiffusion using Southern Biotechnology Associates immunoreagents.

## D. SCREENING PROCEDURE

Eighteen days following fusion, supernates were removed from wells with confluent growth. Samples were tested for antibody binding to autologous squamous carcinoma cell tissue using the following procedure.

1. Five Micron frozen tissue sections are placed on formal-gelatin coated slides prepared by dipping slides in an aqueous solution containing 1.0% formalin and 0.5% gelatin and air drying them.

2. The slides are washed once for 30 minutes at 37°C and once for 30 minutes at room temperature in phosphate buffered saline (PBS), pH 7.2, containing 0.1% bovine serum albumin (BSA).

3. The slides are dipped once in PBS/BSA in a coplin jar and covered with 50-70 microliters of normal goat serum (NGS) at a 1:10 dilution and incubated at room temperature for 20-30 minutes in a moist chamber.

4. The slides are then covered with 5-70 microliters of an appropriate dilution of the cell culture supernatant being screened for antibody activity, and incubated for 20-30 minutes at room temperature.

5. After removing the excess supernatant, the slides are dipped in a coplin jar of PBS/BSA and covered with 50-70 microliters of an appropriate dilution of fluorescein isothiocyanate labelled goat anti-(human Ig) immunoglobulin (TAGO) and incubated for 20-30 minutes at room temperature in the dark.

6. The slides are washed by dipping in a coplin jar with PBS,BSA, dipped once in deionized water and covered with glass coverslips with PBS,glycerine (1:10).

7. The slides are examined under a fluorescent microscope to determine if tumor cells are highlighted by fluorescence.

Both normal and malignant cells are present in the autologous tissue on each slide. In examining the fluorescence shown on each slide, the area of antibody binding (fluorescing area) is identified and recorded, distinguishing hybridomas yielding cell surface binding antibodies, cytoplasm binding antibodies, and nuclear binding antibodies specific for both normal and malignant squamous cells. Hybridomas yielding antibodies which bind to broadly both normal and squamous cells were discarded.

In this manner the wells were screened, and wells showing highly tumor associated antibody and normal cell specific activities were selected for expansion and further characterization. Cells from the selected wells were expanded in HAT medium, replated, incubated and rescreened using standard cloning procedures until wells containing single clones of hybridoma cells which were producing tumor specific antibodies were obtained.

The 17.13 antibody obtained was characterized by immunoglobulin classification, cell binding specificity, normal non-squamous cell binding patterns. The monoclonal antibody was class IgM having binding characteristics summarized as set forth above.

Example 2

The following specific case studies illustrate that the 17.13 antibody distinguishes pre-cancerous from benign cells and dysplasia transformation in the head and neck (including the oral cavity) and in the cervix.

A) Head and Neck Region

Case No. One:

A squamous cell carcinoma of the cheek was surgically resected on January 17, 1984. Pathology confirmed the presence of an infiltrating poorly differentiated SCC. Tissue specimens representing tumor area and adjacent margin area were stained by hemotoxylin and eosin (H&E) and 17.13 antibody. H&E staining established tumor area is cancerous and adjacent margin area is normal. 17.13 antibody stained adjacent margin area brightly (3 + staining); 17.13 antibody also stained adjacent margin area brightly (3 + staining) with full thickness of all epithelial layers stained, indicating the H&E defined "normal" margin is "cancerous". On March 18, 1984, the patient developed recurrence with lymph node involvement. No margin specimens was submitted at surgery. Patient died of recurrence of SCC in June 1984.

Case No. Two:

A focal squamous cell carcinoma and leukoplakia in the tongue was surgically removed on July 19, 1984. Pathology confirmed the right lateral tongue lesion is squamous cell carcinoma-in situ with a single focus of microinvasion (i.e., 0.01mm from basement membrane). The margins of resection appeared free of in situ or infiltrating carcinoma by H&E staining. The surgeon and pathologist felt that the condition does not warrant more extensive surgery, especially since this is an early invasion with clear surgical margins of excision. 17.13 antibody was used to stain the most distant margin specimen from the lesion and showed strong bright staining of all layers of this epithelium. H&E indicated the epithelium looked normal to mild dysplasia. The patient later developed new lesion at the site of suturing. The clinician believes this is a recurrence of SCC, but clinical presentation does not warrant more surgery yet. The patient is currently being followed every three months.

Case No. Three:

A squamous cell carcinoma of the larynx was surgically removed in May, 1982. Patient was under x-ray radiation therapy to the neck for six weeks. In December, 1982, SCC recurrence in the left side of the neck was treated by x-ray. In May, 1983 more surgery was done. During May, 1982 surgery, no specimen was available for staining by 17.13 antibody. The May 1983 surgical tumor and margin specimen was stained with 17.13 antibody. 17.13 antibody stained the frank tumor area brightly. The surgical margin area was not stained by 17.13 antibody except the basal cell layers. H&E also indicated the surgical margin area is morphologically normal. The patient is alive today, no recurrence of SCC was observed.

Case No. Four:

A mucoepidermoid squamous cell carcinoma of the tongue was surgically resected in July 1983. H&E diagnosis indicated SCC in situ and invasive moderately differentiated SCC. Normal margin serial specimens showed mild, moderate dysplasia into carcinoma in situ merging into invasive SCC. 17.13 antibody stained frank malignancy with bright and strong staining. All margin specimens were also stained positively by 7.13 antibody. Patient developed squamous cell carcinoma recurrence in the margin area in April 1984.

Case No. Five:

An invasive keratinizing squamous cell carcinoma involving left piriform sinus was surgically resected in June 1983. H&E diagnosed surgical margin is morphologically normal and free of tumor. 17.13 antibody stained tumor area with bright cytoplasmic stain. 17.13 antibody stained only the basal cell layer of the H&E defined normal margin specimen indicating the margin is normal and safe. The patient is alive and has no SCC recurrence.

B) Cervical Region

Cervical intraepithelial neoplaisa (CIN) were treated conventionally by surgical resection.

The following are two indirect line of results with 17.13 antibody.

(1) Cervical dysplasia confirmed by Papanicolaou procedure (pap-smear)Among 90 cervical CIN I's, 17.13 antibody stained 30 of them. (30/90 = 33%)

Among 55 cervical CIN II's, 17.13 antibody stained 19 of them. (19/55 = 35%)

Among 27 cervical CIN III's, 17.13 antibody stained 9 of them. (9/27 = 33%)

Summary:

Since not all cervical dysplasias will progress to develop into squamous cell carcinoma, 17.13 antibody only stained a fraction of the dysplasia population.

(2) Cervical dysplasia confirmed by biopsyAmong 23 cervical CIN I's, 17.13 antibody stained 6 of them. (6/23 = 26%)

Among 21 cervical CIN II's, 17.13 antibody stained 5 of them. (5/21 = 24%)

Among 31 cervical CIN III's, 17.13 antibody stained 12 of them. (12/31 = 39%)

Summary:

Since the Papanicolaou procedure (pap-smear) identifies cervical dysplasia may not always be true clinical dysplasia, biopsy is a more accurate method to identify the presence of cervical dysplasia. 17.13 antibody only stained a fraction of biopsy proven cervical dysplasia. Not all biopsy confirmed cervical dysplasia were stained by 17.13 antibody.

C) Oral Cavity Region

The following indirect evidence all use MAb 17.13 antibody.
Among 6 confirmed oral dysplasia, the 17.13 antibody stained 5 of them (5/6 = 83%)

Among 4 confirmed leukoplakia, the 17.13 antibody stained 1 of them. (2/4 = 50%)

Among 6 confirmed lichen planus, the 17.13 antibody stained 5 of them. (5/6 = 83%)
Among three proliferating verrucous leukoplakia, the 17.13 antibody stained 3 of them (3/3 = 100%) one of the three patients has developed invasive squamous cell carcinoma in the oral cavity.

Among 8 biopsy obtained margin specimens, the 17.13 antibody stained 4 of them. (4/8 = 50%).

Summary:

The 17.13 antibody stained only a fraction of oral mucosal lesions. One patient stained positive by 17.13 antibody has progressed to squamous cell carcinoma.

## Example 3

The following Table 2 is a summary of a retrospective staining study using the 17.13 antibody in the manner set forth above.

## Example 3

**TABLE 2**

## RETROSPECTIVE 17.13.C1 ANTIBODY STAINING RESULTS

| CASE # | Bx DATE | PATH # | Bx SITE | Dx | 17.13 ANTIBODY STAINING OF EPITHELIAL COMPONENT |
|---|---|---|---|---|---|
| (1) | 05-17-82 | 82-1351 | Rt.Tongue | SCCa | 2-3+, Homogeneous |
| | 10-26-82 | 32-2883 | Rt.Tongue | Benign Focal Keratosis | 2-3+, Variable |
| | 12-07-82 | 82-3296 | Rt.Tongue | Benign Focal Keratosis | 2+, Homogeneous |
| follow-up: | 09-30-86 | 86-3135 | Rt.Tongue | Focal Keratosis | Not Done |
| (2) | 05-21-86 | 86-1680A | Floor of Mouth | SCCa | 3+, Homogeneous |
| | 05-21-86 | 86-1680B | Lt. Oropharynx | Inflammatory Fibrous Hyperplasia | 2-3+, Homogeneous |
| (3) | 12-19-75 | 75-2440C | Tongue | Epithelial Hyperplasia | 2+, Focal |
| | 02-09-76 | 76-0293 | Tongue | Epithelial Hyperplasia | 1-2+, Variable |
| | 02-23-77 | 77-522A | Tongue | Epithelial Hyperplasia | 2-3+, Variable |
| | 02-23-77 | 77-522B | Tongue | Epithelial Hyperplasia | 2-3+, Variable |
| | 09-12-77 | 77-2256 | Tongue | SCCa | 2-3+, Homogeneous |
| (4) | 10-26-71 | 71-1203 | Lower Lip | Leukoplakia c mild Atypia | 1-3+, Variable |
| | 06-27-78 | 78-1650 | Lower Lip | Benign Candidiasis | 2-3+, Variable |
| | 12-09-80 | 80-2960 | Lower Lip | SCCa | 2-3+, Homogeneous |
| | 12-06-83 | 83-3411 | Lower Lip | Dysplasia, Clinical Ca | 1-3+, Variable |

TABLE 2 (Continued)

STAINING

| CASE # | Bx DATE | PATH # | Bx SITE | Dx | OF EPITHELIAL COMPONENT |
|---|---|---|---|---|---|
| (5) | - | 84-2298B | Labial Mucosa | CIS, suspect invasion | 1-3+, Variable |
| | - | 84-3197 | Labial Mucosa | Moderate to severe | |
| | | | | Dysplasia | 1-3+, Variable |
| | - | 85-2377 | Labial Mucosa | Dysplasia | 2-3+, Variable |
| | - | 86-0551 | Labial Mucosa | Severe Dysplasia | 3+, Focal |
| (6) | 03-04-80 | 80-0534 | Lower Lip | Hyperkeratosis with | |
| | | | | Epith. Dysplasia | 1-3+, Variable |
| | 07-01-80 | 80-1525A | Lower Lip | Atypical Verrucous | |
| | | | | Hyperplasia | 2-3+, Variable |
| | 01-14-86 | 86-0125 | Lower Lip | WD SCCa | 2-3+, Homogeneous |
| (7) | 10-23-81 | 81-2797A | Buccal Mucosa | Phogenic Granuloma | 2-3+, Variable |
| | 10-23-81 | 81-2797B | Buccal Mucosa | Leukoplakia c Atypia | 2-3+, Variable |
| | 05-33-83 | 83-1268 | Buccal Mucosa | Moderate to Severe | |
| | | | | Dysplasia | 1-3+, Variable |
| | 06-21-83 | 83-1804A | Buccal Mucosa | MD-SCCa | 3+, Homogeneous |
| (8) | 07-06-84 | 84-1931 | - | PVL, Grade 3 | 2-3+, Variable |
| | 05-27-86 | 86-1740 | - | PVL, Grade 5 | 2-3+, Variable |
| (9) | 09-10-85 | 85-2818 | - | Benign Mucositis | 1-3+, Variable |
| follow-up: | 09-30-86 | 86-1667 | - | Dysplasia | Not Done |

CONTROLS:

| | | | | | |
|---|---|---|---|---|---|
| Normal Tongue | - | | - | Normal | ±/trace, Focal |
| Tumor | - | | Nasopharynx | MD-SCCa | 2-3+, Homogeneous |

Claims

1. A monoclonal antibody or antibody fragment, specific for a site on an antigen associated with the intra-cellular portion of human basal cells and human malignant squamous carcinoma cells.

2. The antibody or antibody fragment of Claim 1 in which said antigen is found totally in the cytoplasm of the cell.

3. A monoclonal antibody or antibody fragment specific for a site on an antigen associated with human basal cells and human malignant squamous carcinoma cells. said antibody being produced by a hybridoma formed by fusing, with other cells, antibody-producing cells derived from an animal immunized with immunogen comprising human cancerous squamous cell tissue or tissue containing basal cells.

4. The antibody or fragment of Claim 3 in which said immunogen also comprises morphologically normal, non-basal human squamous ephithelial cells.

5. The antibody or fragment of Claims 3 or 4 in which said immunogen also comprises dysplastic, non-basal human squamous epithelial cells.

6. The antibody or fragment of Claims 1 or 3 which does not recognize at least 98 % of specimens comprising morphologically normal, non-basal, human epithelial cells.

7. The antibody or fragment of Claims 1 or 3 which recognizes at least 1 % of specimens comprising morphologically normal, non-basal, human squamous epithelial cells.

8. The antibody or fragment of Claim 3 also specific for a site on an antigen associated with at least some morphologically non-tumor, non-basal, human squamous epithelial cell specimens.

9. A monoclonal antibody, or antibody fragment, which is specific for a site on an antigen associated with morphologically normal, non-basal, human squamous epithelial cells which are pre-cancerous but not associated with those cells which are not pre-cancerous.

10. The antibody or fragment of Claim 8 in which said antigen is found in the cytoplasm of said morphologically normal, non-basal, human squamous epithelial cells.

11. The antibody or fragment of Claim 1 also specific for a site on an antigen associated with at least some dysplastic, non-basal, human squamous epithelial cells.

12. The antibody, or antibody fragment, of Claim 11 in which said antigen is found in the cytoplasm of said dysplastic, non-basal, human squamous epithelial cells.

13. A monoclonal antibody or antibody fragment which is specific for a site on an antigen of dysplastic, non-basal, human squamous epithelial cells which are pre-cancerous but not found in those cells which are not pre-cancerous.

14. The antibody or antibody fragment of Claim 13 in which said antigen is found in the cytoplasm of said dysplastic, non-basal human squamous epithelial cells.

15. The antibody or antibody fragment of Claim 1, 3, 9, 11 or 12 in which said antigen site is a component of a macromolecule which contains protein.

16. The antibody, or antibody fragment, of Claims 1 or 3 in which said antigenic site is a component of a macromolecule which contains protein and is stable in the presence of non-ionic detergent Triton X-100.

17. The antibody or antibody fragment of Claims 1 or 3 in which said antigen appears as bright diffuse and bright fibrillar homogeneous by immunofluorescence or immunoperoxidase staining.

18. The antibody or antibody fragment of Claims 1 or 3 in which said antigen resides in the cytoskeletal elements of intermediate filaments by electron microscopic indirect colloidal gold particles (10 nm) staining.

19. The antibody or antibody fragment of Claims 1, 3, 9, 11 or 13 in admixture with at least a second monoclonal antibody specific for a second site on a second antigen associated with a human basal cell and a human malignant squamous tumor cell and binding fragments of said second antibody specific for said antigenic site.

20. The antibody or antibody fragment of Claim 19 in which said second antigen is found in the cytoplasm of a human basal cell.

21. The antibody or antibody fragment of Claim 1 which is 17.13. C1. 10 or antibody having functional equivalency with said antibody.

22. The antibody or antibody fragment of Claim 1, 3, 9, 11 or 13 of the IgG or IgM class.

23. The antibody or antibody fragment of Claim 1 or 3 which does not bind to fibroblast cells, cells in connective tissue, blood vessels, lymphatic vessels, or red or white blood cells.

24. The antibody or antibody fragment of Claims 1 or 2 which reacts substantially homogeneously by immunofluorescence with said human malignant squamous cancer cells.

25. A diagnostic test kit comprising the monoclonal antibody or antibody fragment of Claims 1 or 3 and a detection system for said monoclonal antibody.

26. The test kit of Claim 25 in which said detection system comprises a conjugate of (a) an immunological substance reactive with said monoclonal antibody or antibody fragment. and (b) a detectable label.

27. The test kit of Claim 26 in which said detection system comprises (a) a first component comprising an immunological substance reactive with said monoclonal antibody or antibody fragment. and (b) a second component comprising a conjugate of (1) a substance reactive with said first component with (2) a detectable label. ·

28. The test kit of Claim 25 in which said detection system comprises a detectible label conjugated with said monoclonal antibody, or antibody fragment.

29. A method for determining the presence of a pre-cancerous condition in morphologically normal, non-basal human squamous epithelial cell specimen comprising:

(a) contacting said specimen with a monoclonal antibody or antibody fragment specific for a site on an antigen associated with specimen if it is pre-cancerous, but not associated with said specimen if it is not pre-cancerous, under conditions for binding of said antibody to said antigenic site to form immune complexes, and

(b) detecting said immune complexes as an indication that said specimen is pre-cancerous.

30. A method for determining the presence of a pre-cancerous condition in dysplastic, non-basal, human squamous epithelial cell specimen comprising:

(a) contacting said specimen with a monoclonal antibody or antibody fragment specific for a site on an antigen associated with specimen if it is pre-cancerous, but not associated with said specimen if it is not pre-cancerous, under conditions for binding of said antibody to said antigenic site to form immune complexes, and

(b) detecting said immune complexes as an indication that said specimen is pre-cancerous.

31. A method for determining the presence of a pre-cancerous or cancerous condition in non-basal, human squamous epithelial cell specimen comprising:

(a) contacting said specimen with a monoclonal antibody or antibody fragment specific for a site on an antigen associated with specimen if it is pre-cancerous or cancerous, but not associated with said specimen if it is not pre-cancerous or cancerous, under conditions for binding of said antibody to said antigenic site to form immune complexes, and

(b) detecting said immune complexes as an indication that said specimen is pre-cancerous or cancerous. ·

32. The method of Claims 29. 30 or 31 in which said antibody, or antibody fragment. also is specific for a site on an antigen associated with human basal cells and human malignant squamous carcinoma cells.

33. A hybridoma cell line which secretes the monoclonal antibodies of Claims 1 or 3.

34. A method for forming the hybridoma cell line of Claim 1 comprising immunizing an animal with immunogen comprising human cancerous squamous cell tissue or tissue containing basal cells, fusing spleen cells from said immunized animal with non-immunoglobulin secreting myeloma cells, and selecting a hybridoma which secretes a monoclonal antibody with said specificity.

NORMAL            ABNORMAL            CANCER

①     ②     ③     ④     ⑤     ⑥     ⑦     ⑧

| NORMAL | ATYPIA | SIGNIFICANT ATYPIA | MILD DYSPLASIA | MODERATE DYSPLASIA | SEVERE DYSPLASIA | CARCINOMA IN SITU | FRANK MALIGNANCY |
|---|---|---|---|---|---|---|---|
| | (ATYPICAL) INCLUDES: HYPERPLASIA, METAPLASIA, HYPERKERATOSIS, ETC. | | IN CERVIX CAN BE CALLED CERVICAL INTRAEPITHELIAL NEOPLASIA (CIN I) | IN CERVIX CAN BE CALLED CIN II | IN CERVIX CAN BE CALLED CIN III | (CIS) | (CANCER) |

FIG. — 1

0 275 705

BOULT, WADE & TENNANT   CONFIRMATORY  27 FURNIVAL STREET

CHARTERED PATENT AGENTS
EUROPEAN PATENT ATTORNEYS

PATENTS DESIGNS & TRADE MARKS

LONDON

EC4A 1PQ

J. S. BUSHELL M.A.(CANTAB)
D. R. HARDISTY PH.D. C.CHEM FRSC.
S. D. MAYES. M.A.(CANTAB) M.I.T.M.A.
M. G. D. BAVERSTOCK. M.A. (OXON)
A. R. G. HORTON. B.SC
G. C. BAYLISS. B.SC.
SUSAN J. ALLARD. M.A.(OXON)
R. E. BIZLEY. M.A.(OXON)
T. B. ALEXANDER. M.A.(CANTAB)
R. E. B. CROSS. B.SC. A.R.C.S.

R. E. SUTHERLAND-HARRIS
D. T. WAYTE
J. H. COUCHMAN. M.A.(CANTAB)

TELEGRAMS & CABLES
BOULT LONDON TELEX

TELEX 267271 BOULT G

FACSIMILE: 01-405 1916

(GROUPS II + III)

TELEPHONES. 01-404 5921
01-242 5071

OUR REF      REB/31497/000

YOUR REF     87311402.9

ENCLOSURES   Informal Drawing
Acknowledgement Form

5th January 1988

Dear Sirs,

European Patent Application No. 87311402.9
INTEK DIAGNOSTICS

As the authorised representative in this case I am writing
to inform you that due to a clerical oversight this
application was filed without its accompanying informal
drawing.  Please find enclosed herewith three copies of this
drawing.  Formal representations will follow in due course.

The background material contained in the drawing would be
well known to the skilled man.

If necessary I request that this correction be made under
Rule 88.

Please acknowledge safe receipt of this letter and of the
enclosures by signing the attached acknowledgement slip and
returning the same to me.

Yours faithfully,


BIZLEY; RICHARD EDWARD
of Boult, Wade & Tennant


D G I - EUROPEAN PATENT OFFICE
P.O. Box 5818
Patentlaan 2
2280 HV Rijswijk (XH)
Holland

| EUROPÄISCHES PATENTAMT<br>Zweigstelle in Den Haag<br>Eingangsstelle | EUROPEAN PATENT OFFICE<br>Branch at The Hague<br>Receiving Section | OFFICE EUROPEEN DES BREVETS<br>Département à La Haye<br>Section de dépôt | ☒ P.B. 5818 - Patentlaan. 2<br>2280 HV RIJSWIJK (ZH)<br>Niederlande/Netherlands/Pays-Bas<br>⌁ Telex 31651<br>☎ (070) 40-2040<br>⌁ BREVPATENT |
| --- | --- | --- | --- |

Bizley, Richard Edward
BOULT, WADE & TENNANT
27 Furnival Street
London EC4A 1PQ
GRANDE BRETAGNE

| | Datum/Date<br>**1 0 FEV. 1988** |
| --- | --- |

| Zeichen/Ref/Réf<br>REB/31497/000 | Anmeldung Nr./Application No./Demande n°//Patent Nr./Patent No./Brevet n°<br>87311402.9- / |
| --- | --- |
| Anmelder/Applicant/Demandeur//Patentinhaber/Proprietor/Titulaire<br>InTek Diagnostics | |

Hiermit teilen wir Ihnen mit, dass Ihrem Antrag gemäss Regel 88 EPÜ vom

_____ auf _____

_____

stattgegeben worden ist. Die Stattgabe erfolgte nur zum Zwecke der Ver-
öffentlichung gemäss Artikel 93 EPÜ.

—————————————————

You are hereby informed that your request pursuant to Rule 88 EPC dated

__1st February 1988__ for __adding Fig. 1 (which corresponds__
__with Fig. 1 of the priority document)__

has been allowed. The request has been accepted only for the purpose of
publication in accordance with Article 93 EPC.

—————————————————

Nous vous informons, que votre requête selon la règle 88 CBE du

_____ pour _____

_____

a été accepté. La requête a été acceptée seulement pour les besoins de la
publication conformément à l'article 93 de la CBE.

EINGANGSSTELLE/RECEIVING SECTION/SECTION DE DEPOT

EPA/EPO/OEB Form 1164 (10.86)